(19) **Europäisches Patentamt European Patent Office Office européen des brevets**

(11) **EP 1 861 420 B1**

(12) **EUROPEAN PATENT SPECIFICATION**

(45) Date of publication and mention
of the grant of the patent:
**25.11.2009 Bulletin 2009/48**

(21) Application number: **06723804.8**

(22) Date of filing: **21.03.2006**

(51) Int Cl.:
*C07K 14/34* (2006.01)       *C12N 15/77* (2006.01)
*C12N 1/20* (2006.01)        *C12P 21/00* (2006.01)

(86) International application number:
**PCT/EP2006/002835**

(87) International publication number:
**WO 2006/100108 (28.09.2006 Gazette 2006/39)**

(54) **FERMENTATION PROCESS FOR THE PRODUCTION OF DIPHTHERIA TOXIN**

FERMENTATIONSVERFAHREN ZUR HERSTELLUNG VON DIPHTERIETOXIN

PROCESSUS DE FERMENTATION SERVANT A LA PRODUCTION DE TOXINE DIPHTERIQUE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **23.03.2005 GB 0505996**

(43) Date of publication of application:
**05.12.2007 Bulletin 2007/49**

(73) Proprietor: **GlaxoSmithKline Biologicals SA**
**1330 Rixensart (BE)**

(72) Inventors:
• **DEHOTTAY, Phillippe, Marc, Helene**
**B-1330 Rixensart (BE)**
• **DESSOY, Sandrine**
**B-1330 Rixensart (BE)**
• **LALOUX, Olivier, Marc, Serge, Ghislain**
**B-1330 Rixensart (BE)**
• **ORVAL, Marc, Roger, Fernand**
**B-1330 Rixensart (BE)**

(74) Representative: **Johnston, Caroline Louise et al**
**GlaxoSmithKline**
**Corporate Intellectual Property (CN925.1)**
**980 Great West Road**
**Brentford**
**Middlesex TW8 9GS (GB)**

(56) References cited:
**EP-A- 0 594 950        WO-A-93/25210**

**WO-A-2006/042542     US-A- 5 917 017**

• FASS R ET AL: "HIGH-YIELD PRODUCTION OF DIPHTERIA TOXIN MUTANTS BY HIGH-DENSITY CULTURE OF C7(BETA)TOX+ STRAINS GROWN IN A NON-DEFERRATED MEDIUM" APPLIED MICROBIOLOGY AND BIOTECHNOLOGY, SPRINGER VERLAG, BERLIN, DE, vol. 43, no. 1, 1995, pages 83-88, XP000914601 ISSN: 0175-7598
• INUI MASAYUKI ET AL: "Metabolic analysis of Corynebacterium glutamicum during lactate and succinate productions under oxygen deprivation conditions." JOURNAL OF MOLECULAR MICROBIOLOGY AND BIOTECHNOLOGY. 2004, vol. 7, no. 4, 2004, pages 182-196, XP009070835 ISSN: 1464-1801
• SHU CHIN-HANG ET AL: "Optimization of L-phenylalanine production of Corynebacterium glutamicum under product feedback inhibition by elevated oxygen transfer rate." BIOTECHNOLOGY AND BIOENGINEERING. 20 JAN 2002, vol. 77, no. 2, 20 January 2002 (2002-01-20), pages 131-141, XP002393996 ISSN: 0006-3592
• HE NING ET AL: "Production of a novel polygalacturonic acid bioflocculant REA-11 by Corynebacterium glutamicum." BIORESOURCE TECHNOLOGY. AUG 2004, vol. 94, no. 1, August 2004 (2004-08), pages 99-105, XP002393997 ISSN: 0960-8524
• MITSUHASHI S. AND KOJIMA Y.: "On the production of the toxin by C. Diphtheriae. III. Effect of gases on the toxin-formation." JAPAN. J. EXP. MED., vol. 22, no. 3, 1952, pages 151-155, XP009070453

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to the field of diphtheria antigens, in particular toxins (including mutant forms of diphtheria toxin, such as CRM197) and fermentation processes for the manufacture of bulk cultures of such antigens.

Diphtheria toxin is a protein exotoxin produced by the bacterium *Corynebacterium diphtheria.* It is produced as a single polypeptide that is readily spliced to form two subunits linked by a disulphide bond, Fragment A and Fragment B, as a result of cleavage at residue 190, 192 or 193 (Moskaug et al Biol. Chem. 264: 15709-15713, 1989. Fragment A is the catalytically active portion and is an NAD-dependent ADP-ribosyltransferase which specifically targets a protein synthesis factor EF-2, thereby inactivating EF-2 and shutting down protein synthesis in a cell.

Immunity to a bacterial toxin such as diphtheria toxin may be acquired naturally during the course of infection, or artificially by injection of a detoxified form of the toxin (toxoid) (Germanier, er, Bacterial Vaccines, Academic Press, Orlando, Fl., 1984). Toxoids have traditionally been made by by chemical modification of native toxins (Lingood et al Brit.J. Exp. Path. 44; 177, 1963), rendering them non-toxic while retaining an antigenicity that protects the vaccinated animal against subsequent challenge by the natural toxin. Alternatively, several mutated diphtheria toxins have been described which have reduced toxicity (US4709017, US4950740).

CRM197 is a non-toxic form of the diphtheria toxin but is immunologically indistinguishable from the diphtheria toxin. CRM197 is produced by *C. diphtheriae* infected by the nontoxigenic phase β197tox- created by nitrosoguanidine mutagenesis of the toxigenic carynephage b (Uchida et al Nature New Biology (1971) 233; 8-11). The CRM197 protein has the same molecular weight as the diphtheria toxin but differs from it by a single base change in the structural gene. This leads to a glycine to glutamine change of amino acid at position 52 which makes fragment A unable to bind NAD and therefore non-toxic (Pappenheimer 1977, Ann Rev, Biochem. 46; 69-94, Rappuoli Applied and Environmental Microbiology Sept 1983 p560-564).

Diphtheria toxoid and a mutant form with reduced toxicity, CRM197, are components in many vaccines providing immunity against *Corynebacterium diphtheriae.* Several combination vaccines are known which can prevent *Bordetella pertussis, Clostridium tetani, Corynebacterium diphtheriae,* and optionally Hepatitis B virus and/or *Haemophilus influenzae* type b (see, for instance, WO 93/24148 and WO 97/00697, WO 02/055105).

Diphtheria toxin and mutant forms including CRM197 have also been used in vaccines as safe and effective T-cell dependent carriers for saccharides. CRM197 is currently used in the *Haemophilus influenzae* type b oligosaccharide CRM197 conjugate vaccine (HibTitre ®; Lederle Praxis Biologicals, Rochester, N.Y.).

Methods of preparing diphtheria toxoid (DT) are well known in the art. For instance, DT may be produced by purification of the toxin from a culture of *Corynebacterium diphtheriae* followed by chemical detoxification, or may be made by purification of a recombinant, or genetically detoxified analogue of the toxin (for example, CRM197, or other mutants as described in US 4,709,017, US 5,843,711, US 5,601,827, and US 5,917,017). *Corynebacterium diphtheriae* is cultured under aerobic conditions. Rappuoli et al (Biotechnology February 1985, p161-163) suggest that pO2 should be regulated at 25% by aerating with a mixture of air and oxygen which is automatically regulated to maintain the desired pO2.

Production of significant quantities of diphtheria toxins such as CRM197 for use in vaccines has been hindered due to low protein abundance. This problem has been addressed previously by introducing further copies of a gene encoding diphtheria toxin or a mutant form into *Corynebacterium diphtheriae* (US 4,925,792; US 5,614,382). Such methods lead to an increase in production of about three-fold. Methods of further improving diphtheria toxin yields in a reproducible manner would be of benefit to allow higher levels of production of these valuable antigens.

Accordingly, the present application provides an improved fermentation process comprising a fermentation step of growing a strain of *Corynebacterium diphtheria* in medium in a fermenter under conditions of agitation sufficient to maintain a homogenous culture and limited aeration such that pO2 within the culture falls to less than 4% for the majority of the fermentation step.

The fermentation takes place under aerobic, but limited aeration conditions such that oxygen is used up as soon as it enters the culture during the majority of the fermentation, i.e. after the initial phase in which the density of *C. diphtheriae* is relatively low and pO2 levels may be higher. The inventors have found that culture under such conditions results in more efficient and/or consistent expression of diphtheria toxin or mutant compared to fermentation methods carried out at higher pO2. The process of the invention is more robust than fermentation at higher levels of oxygen, and allows yields of diphtheria toxin to remain high even when the culture medium contains added iron or when complex raw materials of variable quality are used.

In a second aspect of the invention, there is provided a process for manufacturing a preparation of diphtheria toxin or mutant thereof comprising carrying out the fermentation process of the invention and isolating diphtheria toxin or mutant thereof from the culture. Although diphtheria toxin and mutants are described herein, it is envisaged that any C. *diphtheriae* antigen may be isolated using the process of the invention.

The use of such a method results in higher yields of diphtheria toxin or mutant, for example CRM197, compared to when 5% pO2 or higher e.g. 20% is maintained.

In a further aspect of the invention there is provided a use of the diphtheria toxin or mutant thereof of the invention in the preparation of a medicament for the treatment or prevention of bacterial disease, particularly C. *diphtheriae* disease.

In a further aspect of the invention there is provided a method of preventing or treating bacterial infection, particularly C. *diphtheriae* infection comprising administration of the pharmaceutical composition of the invention to a patient.

**Description of the figures**

Figure 1 - Graphs showing the oxygenation profile and its use in determining the KLa of a fermentation. Panel A shows the time course of oxygenation following a shift from nitrogen to air. Panel B shows a plot of In(100-pO2) against time which allows the assessment of KLa by determining the gradient of the line.

Figure 2 - Overview of a fermentation process for *C. diphtheriae.*

Figure 3 - Graph showing the typical kinetics of growth of a culture of C. *diphtheriae.* The line with circular markers show the OD at 650nm after various times of culture. The line marked with diamonds shows the pH of the culture.

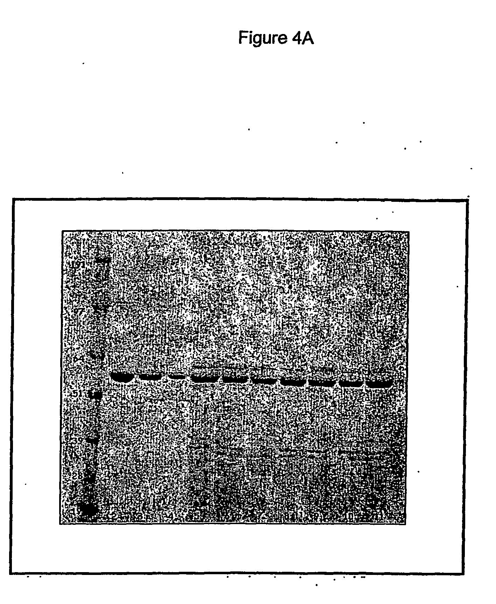

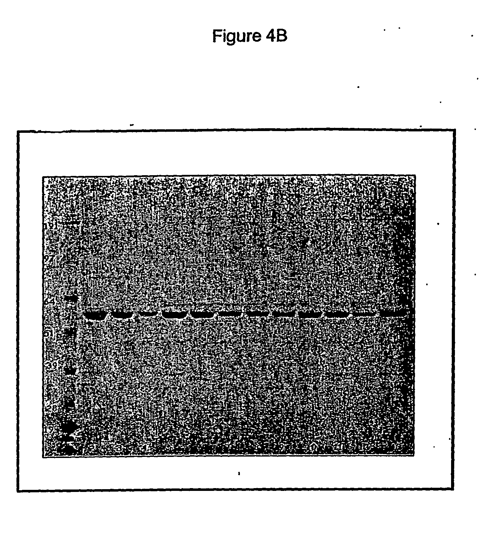

Figure 4 - SDS-PAGE gels of culture supernatants. Lane 1 - molecular weight markers, lane 2 - 1$\mu$g CRM197 standard, lane 3 - 0.5$\mu$g CRM197 standard, lane 4 - 0.25$\mu$g CRM197 standard, lanes 5-11, supernatants from *C. diphtheriae* fermentations. Gel A shows the supernatants from CDT082 in lane 5, CDT198 in lanes 6-8 (the supernatant was removed at 22.5 hours for lane 6, 24 hours for lane 7 and 28 hours for lane 8), CDT199 in lanes 9, 10 and 11 (the supernatant was removed at 22 hour 45 minutes in lane 9, 24 hours 45 minutes in lane 10 and after subsequent microfiltration and filtration in lane 11). Gel B shows supernatants from CDT082 in lane 5, from CDT205 in lanes 6-9 (lane 7 after 21 hours 43 mins of fermentation, lane 8 after 23 hours fermentation, lane 9 after 24 hours fermentation) and from CDT206 in lanes 10-13 (lane 10 after 22 hours 10 mins of fermentation, lane 11 after 23 hours 49 minutes of fermentation, lane 12 after 24 hours 30 mins of fermentation, lane 13 after microfiltarion and filtration).

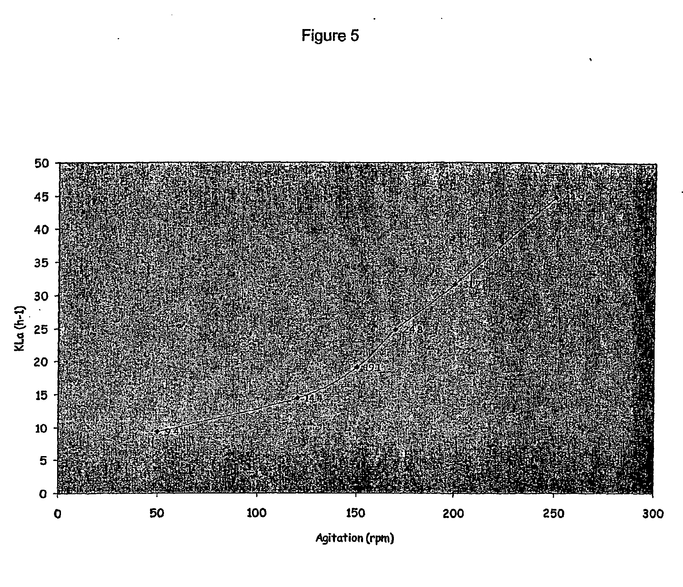

Figure 5 - Graph showing the KLa of a 150 litre fermenter at different agitation speeds under aeration conditions of 23 litres per minute.

**Detailed description of the invention**

The terms "comprising", "comprise" and "comprises" herein are intended by the inventors to be optionally substitutable with the terms "consisting of", "consist of" and "consists of", respectively, in every instance.

One aspect of the invention is a fermentation process comprising a fermentation step of growing a strain of *Corynebacterium diphtheria* in medium in a fermenter under conditions of agitation sufficient to maintain a homogenous culture (for example sufficient to produce a mixing time of less than 30, 20, 15,10, 9, 8, 7, 6, 5, 4, 3, 2 or 1 seconds) and limited aeration such that pO2 within the culture falls to less than 5%, 4%, 3%, 1% or 0.5% for the majority of the fermentation step. In a preferred embodiment, the pO2 falls to approaching zero, preferably for the majority of the fermentation step.

For example, the pO2 within the culture falls to less than 5%, 4%, 3%, 1% or 0.5% from the time when the *Corynebacterium diphtheria* has grown to a density sufficient for it to consume most of the oxygen as soon as the oxygen enters the culture (the latency phase, for example from at least 1, 2, 3, 4, 5 or 6 hours after the start of fermentation), until the point in the fermentation when the pO2 concentration rises again, close to the end of the fermentation step (for example 16, 18, 20, 22 or 24 hours after the latency phase).

Fermentation typically ends and the culture is harvested when pO2 rises above limited aeration conditions. It should be noted that under different inoculation conditions, for example where the fermentor is inoculated with a much larger culture of C. *diphtheriae,* limited aeration conditions commence from shortly after the start of fermentation (for example 1, 5, 10, 20, 30, 40 or 60 minutes after the start of fermentation).

A 100% pO2 is the amount of oxygen present when the medium (in the absence of a culture) is saturated with oxygen following bubbling compressed air through the medium at 34.5°C and pressure of 0.5bar. For a 150Litre fermentor, the aeration rate and agitation speed should be set at 23 litres/min and 240rpm, whereas for a 20 Litre fermentor, the aeration rate and agitation speed should be set at 3 litres/min and 300rpm. It may be set as the amount of oxygen present in a fully aerated fermentation medium prior to inoculation.

A homogenous culture is a culture in which the bacteria are evenly dispersed throughout the fermenter such that at least 3, 4, 5, 6, 7, 8, 9 or 10% of the bacteria are present in the uppermost 10% of the culture medium.

A fermentation step is defined as the step in which *Corynebacterium diphtheriae* is cultured within the fermenter. The fermentation step commences with the introduction of the preculture into the fermenter and ends when, under the limited aeration conditions described herein, the pO2 eventually increases to above 10%. The fermentation step typically lasts for over 12, 14, 16, 18, 20, or 24 hours, for example between 16 and 40 hours, or for example between 22 and 28 hours.

Agitation is optionally by stirring the culture in the fermenter but may be by any other suitable means, for example by agitation, vibromixer and/or gas bubbling. Agitation is sufficient to produce a mixing time for the culture of less than 20, 15, 10, 8, 7, 6, 5, 4, 3, 2 or 1 seconds.

A mixing time of a culture can be measured in a glass fermentor. It is the time taken after the introduction of a coloured aqueous solution for the coloured aqueous solution to be evenly dispersed throughout the culture medium.

A fermenter is any apparatus suitable for the industrial production of bacterial cultures. However this term does not include culture flasks which are typically used for growth of bacteria on a smaller scale.

The majority of the fermentation step is defined as a time of more than 50%, 60%, 70%, 80% or 90% of the total length of the fermentation step. The fermentation is typically under limited aeration conditions for 12, 14, 16, 18, 20, 21, 22, 23, 24, 25, 26 or 28 hours.

Limited aeration describes aeration conditions which allow the C. *diphtheriae* to use aerobic respiration and yet limits the amount of oxygen available such that, after the culture has increased in density (for instance after at least 1, 2, 3, 4, 5, 6, 7, 8, 9 or 10 hours of fermentation) oxygen is consumed very shortly after entering the culture so that the pO2 is less than 5, 4, 3, 2, 1 or 0.5%. It should be noted that by increasing the quantity of culture used to inoculate the fermentor, the limited aeration conditions could be achieved very shortly after inoculation (for example after 1, 5, 10, 20 or 30 minutes after start of fermentation).

Such limited aeration conditions lead to robust expression of a toxin such as diphtheria toxin or mutants thereof.

A pO2 falling to approaching zero is achieved by the rate of aeration and agitation being such that oxygen introduced into the culture is used up by the culture for respiration soon after its introduction into the culture so that despite aeration of the culture, the pO2 is read as zero or close to zero on an oxygen monitor.

During the fermentation step, the pO2 will start at a higher level for a given setting of agitation and rate of aeration. This is because the density of bacteria in the culture is low at the start of the fermentation step and increases during the fermentation step. A period of time (for example, up to 1, 2, 3, 4, 5, 6, 7, 8, 9 or 10 hours) is typically required before the pO2 falls to less than 5%. From this point onwards, the pO2 remains below 5, 4, 3, 2, 1 or 0.5%, preferably at a level approaching zero until close to the end of the fermentation step for instance till the harvesting of the fermenter.

Optionally, the fermentation step is carried out at constant KLa throughout the fermentation step. Alternatively, the fermentation step is carried out at at one or more KLa such that limited aeration is achieved at the KLa values present during the majority (at least 50%, 60%, 70%, 80%, 90%, 95%) of the fermentation step.

KLa is a measure of the rate at which oxygen enters the culture. The higher the KLa, the greater the rate at which oxygen is introduced into the culture. Several factors including the medium volume and composition, agitation, aeration, pressure, temperature and the position and characteristics of mobile parts of the fermenter will influence the KLa of a particular fermentation step.

Typically, oxygen is introduced into the fermentation culture by bubbling compressed air through the culture. Where different concentrations of oxygen are present in the air introduced into the culture, the flow rate should be adapted to take account of this. For instance, where a supply of 100% oxygen is introduced into the culture, the flow rate would be correspondingly lower. Where gas containing less oxygen than air is introduced into the culture, a higher flow rate could be applied.

KLa can be measured using the method described in Example 1. The method involves setting up the fermenter with the conditions of medium volume, temperature, pressure, agitation and aeration for which the KLa is to be measured, gassing out by replacing the air with nitrogen gas, gassing in by restoring air aeration and measuring the rate at which pO2 returns to its steady state level.

$$\ln(100 - pO2) = -KLa. \, T + C$$

By plotting ln (100-pO2) against time, the gradient (or angular coefficient) of the line is - KLa.

The KLa of a fermentation step is influenced by a number of factors including the amount of agitation of the culture and the aeration rate of the culture. A constant KLa may be maintained while for instance decreasing the agitation of the culture and increasing the aeration rate or vice versa. However, in an embodiment, both the agitation of the culture and the aeration rate are constant during the fermentation step.

The fermentation step is carried out, for example, at a KLa of between 10-200h-1, 10 - 150 h-1, 10 - 100 h-1, 10-80 h-1, 10-50 h-1, 10-40h-1, 10-30 h-1, 20-150 h-1, 20-100 h-1, 20-50 h-1, 20-60 h-1, 20-80 h-1, 20-30 h-1, 20-40 h-1, 30-60 h-1, 60-80 h-1, 60-150 h-1 or 60-200 h-1.

The KLa of the fermentation process of the invention may differ, depending on the size of the fermentation culture. For cultures of 10-30 litres, a KLa of 10-30 h-1, 15-30, 20-30 or 22-28 h-1 can be used. For cultures of 30-250 litres, a KLa of 30-60 or 40-50 h-1 can be used. For cultures of 250-800 litres a KLa of 30-50, 40-50, 40-60, 30-60, 30-80 or 60-150 h-1 can be used. For cultures of 800-3000 litres a KLa of 30-50, 40-50, 40-60, 30-60, 30-80, 60-150 or 60-200 h-1 can be used.

For a fermentation culture size of 10-30 litres, a KLa of 10-30 h-1 is achieved for example by using an airflow or aeration rate of 1-5 litres/min and an agitation speed of 200-400rpm, for example an aeration rate of 2-4 litres/min and an agitation speed of 250-350rpm.

For a fermentation culture of 30-250 litres, a KLa of 30-60 h-1 is achieved for example by using an airflow rate of 15-25 litres/min and an agitation speed of 150-250 rpm, for example by using an airflow rate of 20-25 litres/min and an agitation speed of 200-250 rpm, for example by using an airflow rate of 15-20 litres/min and an agitation speed of 200-250 rpm.

The pH of the culture of C. *diphtheriae* in CY medium during the fermentation step depends on the conditions of aeration and agitation of the culture (Nikolajewski et al J. Biological Standardization, 1982, 10; 109-114). At the start of the fermentation step, the pH of the CY medium is 7.4. In the case of low aeration or KLa, the pH drops to around 5. In the case of high aeration, the pH increases up to around 8.5. In one embodiment of the invention, the C. *diphtheriae* is cultured in CY medium or SOC medium (Sambrook J et al 1989, Molecular Cloning: A Laboratory Manual, Cold Spring Harbor Laboratory Press, Cold Spring Harbor, NY) or similar media. The pH within the fermenter may be held between 7.0 and 7.8, by the degree of aeration, optionally without requiring addition of acid or base.

The process of the invention can be used with any strain of *Corynebacterium diphtheriae.* Such strains may produce wild type diphtheria toxin, fusion proteins including diphtheria toxin or fragment thereof (e.g. those disclosed in US 5863891) or mutant forms or fragments of diphtheria toxin, preferably those which have reduced toxicity. Examples of such mutant toxins are CRM176, CRM 197, CRM228, CRM 45 (Uchida et al J. Biol. Chem. 218; 3838-3844, 1973); CRM 9, CRM 45, CRM102, CRM 103 and CRM107 and other mutations described by Nicholls and Youle in Geneticaly Engineered Toxins, Ed: Frankel, Maecel Dekker Inc, 1992; deletion or mutation of Glu-148 to Asp, Gln or Ser and/or Ala 158 to Gly and other mutations disclosed in US 4709017 or US 4950740; mutation of at least one or more residues Lys 516, Lys 526, Phe 530 and/or Lys 534 and other mutations disclosed in US 5917017 or US 6455673; or fragment disclosed in US 5843711. In an embodiment, the strain of C. *diphtheriae* produces CRM197.

In an embodiment, the following strains of C. *diphtheriae* are used in the processes of the invention; ATCC39255, ATCC39526, ATCC11049, ATCC11050, ATCC11051, ATCC11951, ATCC11952, ATCC13812, ATCC14779, ATCC19409, ATCC27010, ATCC27011, ATCC27012, ATCC296, ATCC43145, ATCC51280 or ATCC51696.

The medium for use in the invention may contain one or more of the following constituents: 5-20 g/L, 10-16g/L or 10g/L casamino acids or casein hydrolysate, 5-20 g/L, 7-15g/L or 9-12g/L soya peptone and/or 10-40g/L, 14-32g/L or 18-22g/L yeast extract.

It is known that iron content of the growth medium can affect the growth of C. *diphtheriae* and influence toxin production (see WO 00/50449). Iron is essential for bacterial growth, however, iron in large concentrations has been shown to inhibit the production of toxin. During the process of the invention, the iron content of the medium has a lower level of 10, 50, 75, 100, 120 or 150 ppb and an upper limit of 200, 300, 400, 500, 600, 700, 800, 900, 1000, 1500, 2000, 3000, 4000 or 5000 ppb. For example, iron concentrations in the medium are: 50-1000ppb, 100-1000ppb, 200-1000ppb, 400-1000ppb, 500-1500ppb, 700-1300ppb, 50-2000ppb, 100-2000ppb, 200-2000ppb, 400-2000ppb, 700-2000ppb, 50-3000ppb, 100-3000ppb, 200-3000ppb, 400-3000ppb, 700-3000ppb, 1000-3000ppb, 1500-3000ppb, 1700-3000ppb, 50-4000ppb, 100-4000ppb, 200-4000ppb, 400-4000ppb, 700-4000ppb, 1000-4000ppb, 1500-4000ppb, 1700-4000ppb or 2000-4000ppb. The iron may be in the form of $Fe^{2+}$ and/or $Fe^{3+}$.

In an embodiment, the process of the invention is sufficiently tolerant to the presence of iron salts in the medium such that no treatment of the medium to remove iron in required before use.

The fermentation step takes place at a temperature suitable for the culture of C. *diphtheriae,* for example 25-45 °C, 25-40 °C, 30-38 °C, or 34-35 °C.

The fermentation step is subject to a large amount of foam production. In order to control foam formation an antifoam agent is optionally added to the fermenter. Optionally a foam probe or mechanical foam breaker is used in the fermentor, for example as well as the antifoam agent.

A second aspect of the invention is a process for manufacturing a preparation of an antigen, for instance, diphtheria toxin or mutant or fragment thereof comprising the steps of carrying out the fermentation process of the invention as described above and isolating the antigen, for example, diphtheria toxin or mutant or fragment thereof from the culture.

The toxicity of the diphtheria toxin is optionally reduced by chemical treatment including treatment with cross-linking reagents to form a toxoid. References to a toxin include toxoids.

The pharmaceutical composition of the invention optionally further comprises additional antigens in a combination vaccine. In an embodiment, antigen(s) to be combined with the diphtheria toxin, mutant or fragment thereof as described above include one or more of tetanus toxoid, whole cell pertussis (Pw), acellular pertussis (Pa) (as described below), Hepatitis B surface antigen, Hepatitis A virus, *Haemophilus influenzae* b polysaccharides or oligosaccharides, neisserial (e.g. *N. meningitidis)* polysaccharides or oligosaccharides, *N. meningitidis* serotype B proteins, optionally as part of an outer membrane vesicle, pneumococcal polysaccharides or oligosaccharides, pneumococcal proteins or any of the antigens listed below. Bacterial polysaccharides may be conjugated to a carrier protein. Diphtheria toxin or toxoid or mutants of diphtheria toxin such as CRM197 or fragments, for example made using a process of the invention, may be used as carrier protein. However other carrier proteins such as tetanus toxoid, tetanus toxoid fragment C, pneumolysin, Protein D (US6342224) may also be used. A given pharmaceutical composition optionally contains multiple polysaccharides or oligosaccharides conjugated to different carrier proteins.

Diphtheria toxin, or mutant thereof, for example CRM197, or fragment thereof made using the process of the invention may be formulated with capsular polysaccharides or oligosaccharides derived from one or more of *Neisseria meningitidis, Haemophilus influenzae* b*, Streptococcus pneumoniae,* Group A Streptococci, Group B Streptococci, *Staphylococcus aureus* or *Staphylococcus epidermidis.* For example, the pharmaceutical or immunogenic composition may comprise capsular polysaccharides derived from one or more of serogroups A, C, W-135 and Y of *Neisseria meningitidis.* For example serogroups A and C; A and W, A and Y; C and W, C and Y; W and Y; A, C and W; A C and Y; A, W and Y; C, W and Y or A, C, W and Y may be formulated with CRM197. In another example, the immunogenic composition comprises capsular polysaccharides derived from *Streptococcus pneumoniae.* The pneumococcal capsular polysaccharide antigens are preferably selected from serotypes 1, 2, 3, 4, 5, 6B, 7F, 8, 9N, 9V, 10A, 11A, 12F, 14, 15B, 17F, 18C, 19A, 19F, 20, 22F, 23F and 33F (most preferably from serotypes 1, 3, 4, 5, 6B, 7F, 9V, 14, 18C, 19F and 23F). A further example would contain the PRP capsular polysaccharides (or oligosaccharides) of *Haemophilus influenzae* type b. A further example would contain the Type 5, Type 8, 336, PNAG or dPNAG capsular polysaccharides of *Staphylococcus aureus.* A further example would contain the Type I, Type II, Type III or PIA capsular polysaccharides of *Staphylococcus epidermidis.* A further example would contain the Type Ia, Type Ic, Type II or Type III capsular polysaccharides of Group B streptococcus. A further example would contain the capsular polysaccharides of Group A streptococcus, optionally further comprising at least one M protein and more preferably multiple types of M protein.

The bacterial polysaccharides for use in the invention may be full length, being purified native polysaccharides. Alternatively, the polysaccharides are sized between 2 and 20 times, for example 2-5 times, 5-10 times, 10-15 times or 15-20 times, so that the polysaccharides are smaller in size for greater manageability. Oligosaccharides typically contain between 2 and 20 repeat units.

Such capsular polysaccharides may be unconjugated or conjugated to a carrier protein such as tetanus toxoid, tetanus toxoid fragment C, diphtheria toxoid or CRM197 (both for example made by the method of the invention), pneumolysin, or Protein D (US6342224). Tetanus toxin, diphtheria toxin and pneumolysin are detoxified either by genetic mutation and/or by chemical treatment.

The polysaccharide or oligosaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Optionally, the cyanate ester is coupled with hexane diamine and the amino-derivatised polysaccharide is conjugated to the carrier protein using heteroligation chemistry involving the formation of the thioether linkage. Such conjugates are described in PCT published application WO93/15760 Uniformed Services University.

The conjugates can also be prepared by direct reductive amination methods as described in US 4365170 (Jennings) and US 4673574 (Anderson). Other methods are described in EP-0-161-188, EP-208375 and EP-O-477508.

A further method involves the coupling of a cyanogen bromide activated polysaccharide derivatised with adipic acid hydrazide (ADH) to the protein carrier by Carbodiimide condensation (Chu C. et al Infect. Immunity, (1983) 245; 256).

In particular examples the diphtheria toxin or fragment of mutant thereof (for example CRM197) is conjugated to 1, 2, 3, 4, 5, 6, 7, 8, 9, 10, 11, 12, 13, 14, 15, 16, 17, 18, 19 or 20 additional antigens of the pharmaceutical composition. In an embodiment, it is conjugated to polysaccharide component(s), for instance bacterial polysaccharides including those listed above.

The pharmaceutical or immunogenic composition of the invention may further comprise additional protein components. It is optionally formulated with antigens providing protection against one or more of tetanus and *Bordetella pertussis* infections. The pertussis component may be killed whole cell *B. pertussis* (Pw) or acellular pertussis (Pa) which contains at least one antigen (preferably two or all three) from PT, FHA and 69kDa pertactin. Certain other acellular pertussis formulations also contain agglutinogens such as Fim2 and Fim 3 and these vaccines are also contemplated for use in the invention. Typically, the antigen providing protection against Tetanus is tetanus toxoid which is either chemically inactivated toxins (for example, following treatment with formaldehyde) or inactivated by the introduction of one or more point mutation(s).

The pharmaceutical or immunogenic composition of the invention optionally comprises pneumococcal proteins antigens, for example those pneumococcal proteins which are exposed on the outer surface of the pneumococcus (capable of being recognised by a host's immune system during at least part of the life cycle of the pneumococcus), or are proteins which are secreted or released by the pneumococcus. For example, the protein may be a toxin, adhesin, 2-component signal tranducer, or lipoprotein of *Streptococcus pneumoniae,* or fragments thereof. Examples of such proteins include, but are not limited to: pneumolysin (preferably detoxified by chemical treatment or mutation) [Mitchell et al. Nucleic Acids Res. 1990 Jul 11; 18(13): 4010 "Comparison of pneumolysin genes and proteins from Streptococcus pneumoniae types 1 and 2.", Mitchell et al. Biochim Biophys Acta 1989 Jan 23; 1007(1): 67-72 "Expression of the pneumolysin gene in Escherichia coli: rapid purification and biological properties.", WO 96/05859 (A. Cyanamid), WO 90/06951 (Paton et al), WO 99/03884 (NAVA)]; PspA and transmembrane deletion variants thereof (US 5804193 - Briles et al.*);* PspC and transmembrane deletion variants thereof (WO 97/09994 - Briles et al); PsaA and transmembrane deletion variants thereof (Berry & Paton, Infect Immun 1996 Dec;64(12):5255-62 "Sequence heterogeneity of PsaA, a 37-kilodalton putative adhesin essential for virulence of Streptococcus pneumoniae*"*); pneumococcal choline binding proteins and transmembrane deletion variants thereof; CbpA and transmembrane deletion variants thereof (WO 97141151; WO 99/51266); Glyceraldehyde-3-phosphate - dehydrogenase (Infect. Immun. 1996 64:3544); HSP70 (WO 96/40928); PcpA (Sanchez-Beato et al. FEMS Microbiol Lett 1998, 164:207-14); M like protein, (EP 0837130) and adhesin 18627, (EP 0834568). Further pneumococcal protein antigens for inclusion in the immunogenic composition are those disclosed in WO 98/18931, WO 98/18930 and PCT/US99/30390.

Examples of Neisserial proteins to be formulated with the immunogenic composition of the invention include TbpA (WO93/0686 EP586266; WO92/03467; US5912336), TbpB (WO93/06861; EP586266), Hsf (WO99/31132), NspA (WO96/29412 Hap (PCT/EP99/02766), PorA, PorB, OMP85 (also known as D15) (WO00/23595), PilQ (PCT/EP99/03603), PldA (PCT/EP99/06718), FrpB (WO96/31618 see SEQ ID NO:38), FrpA or FrpC or a conserved portion in common to both of at least 30, 50, 100, 500, 750 amino acids (WO92/01460), LbpA and/or LbpB (PCT/EP98/05117; Schryvers et al Med. Microbiol. 1999 32: 1117), FhaB (WO98/02547 SEQ ID NO: 38), HasR (PCT/EP99/05989), lipo02 (PCT/EP99/08315), MltA (WO99/57280) and ctrA (PCT/EP00/00135). Neisserial proteins are optionally added as purified proteins or as part of an outer membrane preparation.

The pharmaceutical or immunogenic composition of the invention optionally comprises one or more antigens that can protect a host against non-typeable *Haemophilus influenzae,* RSV and/or one or more antigens that can protect a host against influenza virus.

Examples of non-typeable *H. influenzae* protein antigens include Fimbrin protein (US 5766608) and fusions comprising peptides therefrom (eg LB1 Fusion) (US 5843464 - Ohio State Research Foundation), OMP26, P6, protein D, TbpA, TbpB, Hia, Hmw1. Hmw2, Hap, and D15.

Examples of Influenza virus antigens include whole, live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof.

Examples of RSV (Respiratory Syncytial Virus) antigens include the F glycoprotein, the G glycoprotein, the HN protein, the M protein or derivatives thereof.

It should be appreciated that antigenic compositions of the invention may comprise one or more capsular polysaccharide from a single species of bacteria. Antigenic compositions may also comprise capsular polysaccharides derived from one or more species of bacteria.

Optionally, the immunogenic composition or vaccine contains an amount of an adjuvant sufficient to enhance the immune response to the immunogen. Suitable adjuvants include, but are not limited to, aluminium salts, squalene mixtures (SAF-1), muramyl peptide, saponin derivatives, mycobacterium cell wall preparations, monophosphoryl lipid A, mycolic acid derivatives, non-ionic block copolymer surfactants, Quil A, cholera toxin B subunit, polphosphazene and derivatives, and immunostimulating complexes (ISCOMs) such as those described by Takahashi et al. (1990) Nature 344:873-875. For veterinary use and for production of antibodies in animals, mitogenic components of Freund's adjuvant can be used.

As with all immunogenic compositions or vaccines, the immunologically effective amounts of the immunogens must be determined empirically. Factors to be considered include the immunogenicity, whether or not the immunogen will be complexed with or covalently attached to an adjuvant or carrier protein or other carrier, route of administrations and the number of immunising dosages to be administered. Such factors are known in the vaccine art and it is well within the skill of immunologists to make such determinations without undue experimentation.

The active agent can be present in varying concentrations in the pharmaceutical composition or vaccine of the invention. Typically, the minimum concentration of the substance is an amount necessary to achieve its intended use, while the maximum concentration is the maximum amount that will remain in solution or homogeneously suspended within the initial mixture. For instance, the minimum amount of a therapeutic agent is preferably one which will provide a single therapeutically effective dosage. For bioactive substances, the minimum concentration is an amount necessary for bioactivity upon reconstitution and the maximum concentration is at the point at which a homogeneous suspension cannot be maintained. In the case of single-dosed units, the amount is that of a single therapeutic application. Generally, it is expected that each dose will comprise 1-100$\mu$g of protein antigen, preferably 5-50$\mu$g and most preferably 5-25$\mu$g. Preferred doses of bacterial polysaccharides are 10-20$\mu$g, 10-5$\mu$g, 5-2.5$\mu$g or 2.5-1$\mu$g. The preferred amount of the substance varies from substance to substance but is easily determinable by one of skill in the art.

The vaccine preparations of the present invention may be used to protect or treat a mammal (for example a human patient) susceptible to infection, by means of administering said vaccine via systemic or mucosal route. These administrations may include injection via the intramuscular, intraperitoneal, intradermal or subcutaneous routes; or *via* mucosal administration to the oral/alimentary, respiratory, genitourinary tracts. Although the vaccine of the invention may be administered as a single dose, components thereof may also be co-administered together at the same time or at different times (for instance if polysaccharides are present in a vaccine these could be administered separately at the same time or 1-2 weeks after the administration of the bacterial protein combination for optimal coordination of the immune responses with respect to each other). In addition to a single route of administration, 2 different routes of administration may be used. For example, viral antigens may be administered ID (intradermal), whilst bacterial proteins may be administered IM (intramuscular) or IN (intranasal). If polysaccharides are present, they may be administered IM (or ID) and bacterial proteins may be administered IN (or ID). In addition, the vaccines of the invention may be administered IM for priming doses and IN for booster doses.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

A further aspect of the invention is a process for manufacturing a pharmaceutical composition comprising a step of making the diphtheria toxin or fragment or mutant thereof (for instance CRM197) using the fermentation process of the invention and combining it with a pharmaceutically acceptable carrier and optionally adding any of the additional antigens mentioned above.

Such a process may further comprise a step of conjugating the diphtheria toxin or fragment or mutant thereof (for instance CRM197) to one or more additional components of the pharmaceutical composition, preferably bacterial polysaccharides or oligosaccharides as described above.

A further aspect of the invention is use of the diphtheria toxin or fragment or mutant thereof (for instance CRM197) of the invention in the preparation of a medicament for the treatment or prevention of bacterial disease, in particular *C. diphtheriae* disease.

A further aspect of the invention is a method of preventing or treating bacterial infection, in particular *C. diphtheriae* infection, comprising administration of the pharmaceutical composition, immunogenic composition or vaccine of the invention to a patient.

The invention is illustrated in the accompanying examples. The examples below are carried out using standard techniques, which are well known and routine to those of skill in the art, except where otherwise described in detail. The examples are illustrative, but do not limit the invention.

## EXAMPLES

### Example 1: Measurement of KLa

In order to measure the KLa of a fermentation step, the fermenter was filled with the desired volume of water and the fermentation parameters (for instance temperature, pressure, agitation and aeration) were applied and the system left to achieve a steady state. The pO2 probe was calibrated at 100%.

The aeration was shifted rapidly to nitrogen gas, while maintaining the same flow rate. The pO2 was followed until pO2 dropped to less than 5%. When this point was reached, the aeration was shifted rapidly to air while maintaining the same flow rate. The pO2 level was followed and recorded at several time points as a percentage of the original steady state 100% level.

KLa was calculated by plotting the log (100-pO2%) against time. The angular coefficient of the linear part of the graph corresponds to -Kla. Typically, only data between 20% and 80% pO2 are considered.

Results

The pO2 readings at various time points are shown in Table 1 below.

Table 1

| Time (seconds) | Time (hours) | pO2 (%) | ln(100-pO2) |
|---|---|---|---|
| 200 | 0.056 | 20 | 4.382026635 |
| 210 | 0.058 | 21.9 | 4.357990057 |
| 220 | 0.061 | 23 | 4.343805422 |
| 230 | 0.064 | 25 | 4.317488114 |
| 240 | 0.067 | 27 | 4.290459441 |
| 250 | 0.069 | 29 | 4.262679877 |
| 260 | 0.072 | 32 | 4.219507705 |

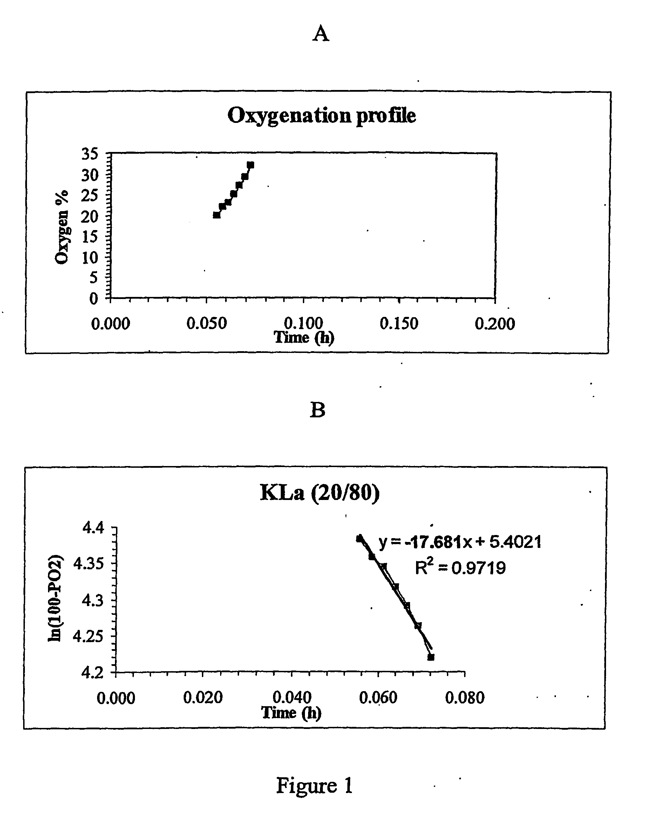

The results were plotted out as shown in figure 1 and the KLa determined from the angular coefficient of the line in Figure 1B.

**Example 2: Fermenation of *C. diphtheriae* strain ATCC 39255 at 150 Litre scale**

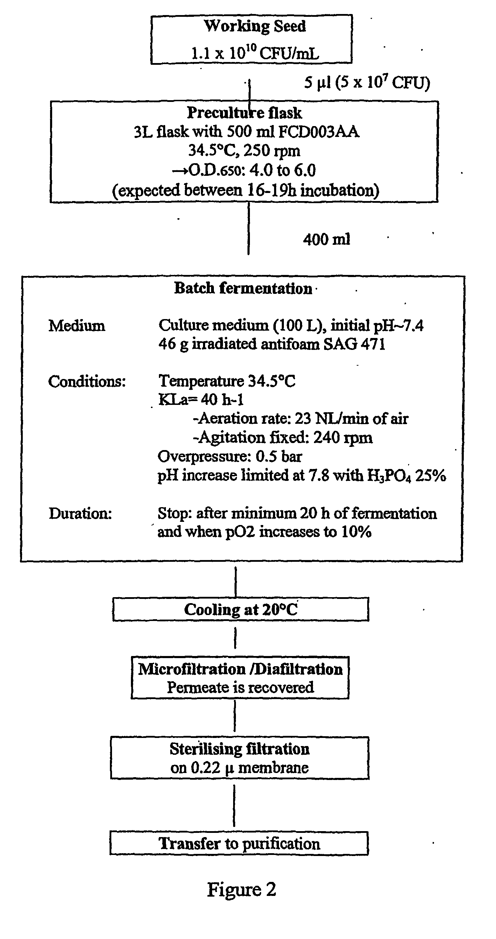

The bacterium used in the preparation of CRM197 (Cross Reacting Material) is a mutant strain of *Corynebacterium diphtheriae* obtained by nitrosoguanidine treatment according to the method of A. Pappenheimer (Nature New Biol. 233:8-11, 1971). It expresses a detoxified diphtheric toxin (aa 52: glycine to glutamic acid mutation). It was obtained from the ATCC where it is referred to as 39255. The general outline of the fermentation process is shown in Figure 2. A working seed containing $1.1 \times 10^{10}$ cfu/m) was withdrawn from the freezer (-70°C) and thawed at room temperature. Immediately after thawing, the vial was vortexed and 250 $\mu$l of the seed are taken with a 1ml syringe with needle.

This volume was injected in 100 ml of sterile saline solution (0.9%). The flask was agitated. Two ml of the suspension were taken with a 2ml syringe with needle and used to inoculate a 3-L erlenmeyer containing 500 mL of the medium described in US4,925,792. The flask was incubated at 34.5°C $\pm$ 0.5°C under 250 rpm agitation speed until the optical density (650 nm) reached 4.0 to 6.0 (after 16 to 19h of incubation).

A 150 litre fermenter was sterilised and 100 L of culture medium were aseptically transferred into the fermenter. The acid bottle was filled with 500 mL $H_3PO_4$ 25 % (V/V); the pH of the medium was initially around 7.4 and was not adjusted.

The fermenter was prepared the day before the inoculation and was kept in stand-by conditions of 34.5°C temperature, 0.5 barg pressure, air flow of 23 N Umin in the headspace and agitation speed 50rpm for 16-20 h, until inoculation.

Prior to inoculation, the fermenter was set to the culture conditions of 34.5°C temperature, 0.5 bar pressure, air flow of 23 N Umin sparged in the medium and agitation speed 240rpm. An agitation of 240 rpm gives a tip speed of 1.76 m/s and a theoretical mixing time of 3.9 seconds. The dissolved oxygen is not regulated, only monitored, the foam control system was switched on and the pH was allowed to reach 7.8 and therefafter maintained by addition of $H_3PO_4$. Prior to inoculation, the p02 probe was set to 100%.

The agitation speed was set at 240 rpm, corresponding to a peripheral speed of 1.76 m/s. The agitation speed of 240 rpm combined with an aeration rate of 23-L/min resulted in a KLa (20-80%, water 30°C, 0.5 bar) estimated at 42 h-1 (see Figure 6).

The fermenter was inoculated through the inoculum port with 400 ml of the seed culture described above.

Fermentation continued until both of the following conditions were met. 20 hours of fermentation are elapsed and dissolved oxygen level had increased to 10%. The total fermentation duration was generally between 22 and 28 h.

At the end of the fermentation, the temperature was changed to a setting of 20°C, the pH regulation was turned off, the air flow was shifted to the headspace in order to limit the foaming, the foam control system was turned off, the other parameters were not changed.

The microfiltration system was connected to the fermenter and when the temperature of the suspension reached 21°C, the microfiltration started. The microfiltration was operated in two phases: a concentration and a diafiltration. During the concentration phase, the parameters were: pressure in: 0.6 bar, pressure out: ~0.1 bar, permeate flow: maintained constant at 2L/min using a calibrated peristaltic pump (the permeate pressure was about 0.3 bar). The suspension was concentrated until one of the following events first happened. Either the inlet pressure reached 0.9 bar or 75 litres of permeate were recovered.

During the diafiltration phase, the following parameters were used: pressure in was kept at the pressure reached at the end of the concentration step (0.9 bar maximum); water was added at 2 litres/min; total water added was 3 volumes of retentate.

The retentate was filtered on a 0.22 μm membrane and stored at +4°C. The stability of the CRM 197 in such suspension was tested after up to 4 days of storage at either +4°C or at room temperature (+20°C<RT<23°C). No degradation and no differences were observed either by ELISA quantification or on SDS-page. A test for confirmation of absence of growth was done on BAB agar incubated at 36°C.

**Results**

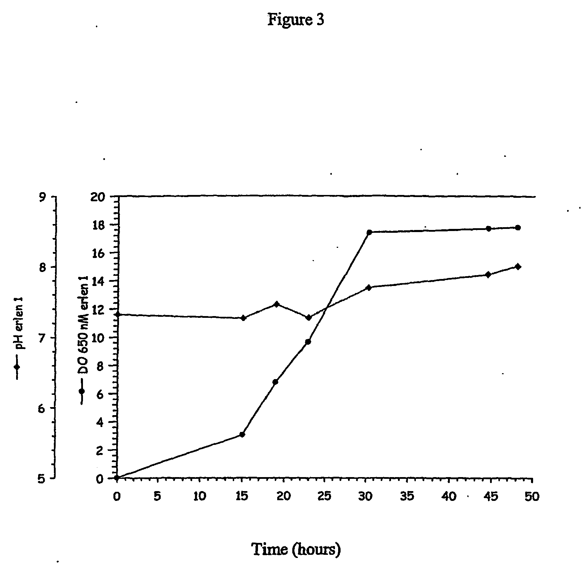

Two fermentations at 150-L scale were carried out using the protocol of fermentation described above (CDT 199 and CDT 206). The conditions of preculture and culture are shown in Tables 2 and 3 and yields of CRM197 are shown in Table 4. Figure 3 shows a graph of the typical kinetics of growth of a preculture. When the O.D. (650 nm) was between 4.0 and 6.0, the culture was clearly in an exponential phase of growth and the pH changed only slightly.

Table 2 Conditions of Precultures

| Culture n° | Preculture duration (H:min) | O.D.650 of preculture | Final pH |
|---|---|---|---|
| CDT199 | 16:44 | 5.88 | 7.30 |
| CDT206 | 17:53 | 4.03 | 7.25 |

Table 3 Conditions of Cultures

| Culture n° | Fermentation duration (h:min) | H3PO4 25% used (g) | Final O.D.650 | Total antifoam quantity needed (g) | CFU estimation (bact/ml) |
|---|---|---|---|---|---|
| CDT199 | 24:45 | 2 | 17.2 | 102 | 4.9 E9 |
| CDT206 | 24:31 | 2 | 13.9 | 119 | Not done |

Table 4 CRM 197 estimation

| Culture n° | Densito. on SDS-page (mg/L) | Elisa (mg/L) |
|---|---|---|
| CDT199 | 89/106 | 138 |
| CDT206 | 92/83 | 116 |

During the fermentation, different phases were observed.
The first phase was characterised by a decrease of the dissolved oxygen until 0% was reached (duration of around 6-7 h). During this phase, the pH remains stable or slightly decreases (by about 0.1 pH unit).
During the second phase the pH increased and reached a plateau at about pH 7.8. At this level, pH regulation was triggered however often no acid at all had to be added under these fermentation conditions. During this plateau of pH, an increase of the dissolved oxygen level above 0% followed by a drop to 0% was observed.
The third phase was characterised by a decrease of the pH to about 7.4.
Finally an increase of pO2 was observed between 22 and 24 h of fermentation. This is the signal for harvest.
The exhaust gases of the fermenter were analysed by mass spectrometer. A typical profile of CO2 production was observed.
The two fermentations presented were prolonged after the signal of harvest in order to estimate the kinetics of CRM 197 production. We observed that the CRM 197 level does not increases after the signal of harvest was reached but there was an increase in foam production. In order to limit the antifoam consumption, it is preferable to stop the fermentation at the signal to harvest. However, the CRM 197 seems not affected by excessive foaming and no degradation occurred.

Microfiltration

Data are shown for the microfiltration of CDT206.
74.3 L of permeate were harvested when the inlet pressure reached 0.9 bars. The outlet pressure was between 0.15 and 0.10 bar throughout the concentration step. The permeate pressure was 0.3-0.4 bar throughout the concentration step and the concentration step lasted for 36 minutes.

For the diafiltration phase, 75-L of water were progressively added (2 Umin) while the permeate was extracted at the same flow rate. The inlet pressure was 0.9 bar at the beginning and dropped to 0.7 bar at the end of the diafiltration. The outlet pressure was stable at 0.1 bar. The duration of the diafiltration step was 39 min.

CRM197 quantification

The level of expression of CRM197 under the low aeration conditions was generally 2-4 fold higher than that achieved under conditions of higher aeration where pO2 was maintained at 5% or higher throughout the fermentation.

Stained SDS page of culture supernatants

SDS-PAGE gels (Figure 4) were run of the culture supematents under reducing condition so that it was possible to detect any degradation bands (after clipping, 2 sub-units of respectively 35 and 23 kD can be detected). They were subsequently stained with Coomassie Blue.

Results

The coomassie stained gels of samples from the two fermentations are shown in Figure 4A (CDT 199) and 4B (CDT 206). The CRM 197 appeared at the expected molecular weight (theoretical MW: 58.4 kD). The CRM 197 was not degraded since no pattern change was seen in samples taken after the signal of end of fermentation (Figure 4A, lanes 9 and 10). The CRM 197 quantity was not affected by the microfiltration and final filtration on $0.22\mu M$ since lane 9 and 11 of Figure 4A show equivalent amounts of CRM197.

Temperature can have a negative effect on CRM stability (Figure 4B lane 12). This sample was taken while the sample valve was warm and the amount of CRM197 present in this sample is reduced.

**Example 3: Fermentation of *C. diphtheriae* strain ATCC 39255 at 20 litre scale**

A method similar to that described in example 2 was used to ferment C. *diphtheriae* except that a 20 litre fermentor was used. The culture was agitated at 300rpm and the air flow was set at 3 litre/minute. No addition of acid or base was required during the fermentation since the pH stayed around neutral throughout the fermentation.

The culture grew to a final OD (650nm) of 18.3. The yield of CRM197 was found to be 103mg/litre as assessed by densitometry on a stained gel.

**Example 4: Fermenation of *C. diphtheriae* strain ATCC 39255 at different scales**

The fermentation process of growing C. *diphtheriae* under conditions of constant KLa can be adapted for use in fermenters of other sizes and different designs. Good yields of CRM197 production were achieved following the conditions of fermenter size, air flow and agitation speed indicated in Table 5. The three 150L scale fermentations were carried out in fermentors of different design.

Table 5

| Scale (L) | Air flow (L/min.) | Agitation speed (rpm) | KLa (h-1) |
|---|---|---|---|
| 20 | 3 | 300 | 22-28 |
| 150 | 23 | 240 | ~42 |
| 150 | 23 | 185 | ~50 |
| 150 | 17 | 200 | ~40 |

As shown in table 5, the KLa was important rather than a specific choice of air flow and agitation speed conditions. Thus a lower air flow could be compensated by a higher agitation speed to result in a similar KLa and the good yields of CRM197 were still achieved. The agitation speed should be sufficient to produce a homogeneous suspension and aeration is limited to maintain a low pO2. N.B. Different fermentors were used for the 20 litre fermentations. The different geometries of the different fermentors led to the range of kLa values shown in table 5.

However, different KLa conditions were optimal for different scales of fermentation. Hence for fermentation in a 20 Litre fermenter, a lower KLa of 22-28 h-1 was optimal.

Optimal KLa conditions are those that allow limited aeration such that the pO2 within the culture falls to low levels. One skilled in the art should easily be able to determine such condtions for a particular size and geometry of fermenter.

**Example 5: Effect of iron concentration on the yield of fermentations at different p02.**

A series of fermentations of C. *diptheriae* strain ATCC 39255 were carried out at 20 litre scale following the method set out in Example 3 so that pO2 was low throughout the majority of the fermentation or at a constant setting of 5% pO2. The amount of $Fe^{3+}$ present was varied between no $Fe^{3+}$ addition, 250ppb addition of $Fe^{3+}$, 500 ppb $Fe^{3+}$ addition and 500ppb $Fe^{2+}$ addition. The yield of CRM197 at the end of fermentation was measured by densitometry of an SDS-PAGE gel. This method tends to give results approximately 20% lower than those achieved by ELISA.

Results

Table 6

| Fermention conditions | CRM197 yield (mg/litre) |
|---|---|
| 5% pO2 medium with no $Fe^{3+}$ addition | 38 |
| 5% pO2 medium with 250ppb $Fe^{3+}$ added | 14 |
| 5% pO2 medium with 500ppb $Fe^{3+}$ added | 18 |
| 5% pO2 medium with 500ppb $Fe^{2+}$ added | 13 |
|  |  |
| Low pO2, constant Kla, medium without $Fe^{3+}$ addition | 100 |
| Low pO2, constant Kla, medium with 250ppb $Fe^{3+}$ addition | 88 |
| Low pO2, constant Kla, medium with 500ppb $Fe^{3+}$ addition | 97 |

As shown in table 6, when C. *diphtheriae* is fermented at 5% pO2, the addition of iron leads to a reduction of yield. However, when the level of pO2 is reduced and the fermentation is carried out under the conditions of low pO2 at constant Kla as described in example 3, higher yields were achieved and the yield was not affected by the addition of $Fe^{3+}$.

**Example 6: Effect of iron concentration on the yield of DT or CRM197 under low aeration conditions**

The range of iron concentration that does not impact expression of CRM197 was determined in microplates. Microplates simulate the limited aeration conditions existing in the fermentation process of the invention.
Culture in microplates was performed in the same medium as was used in the fermentations described above ( in a medium similar to CY medium). $Fe^{3+}$ was added from a stock solution of $FeCl_3.6H_2O$ at 1 g/l $Fe^{3+}$ ion.
The wells of microtitre plates were filled with the medium and were inoculated at 8 E5 bact/mL.
The microtitre plates were incubated at 34.5°C under agitation of 250 rpm for 46h in the case of both *Corynebacterium diphtheriae* expressing CRM197 and *Corynebacterium diphtheriae* expressing Diphtheria Toxin.
The samples were filtered through a 0.22 $\mu$m filter.
The expression was measured by densitometry method on SDSpage (XT Criterion 4-12% bis tris from BioRad) colored with Coomassie blue (Gelcode blue stain from Pierce). The reference used for the quantification was the diphtheria toxin CRM mutant of List Biological Laboratories INC, introduced at different concentrations on the gel.

Results

Table 7 shows the expression of CRM197 under different iron concentrations for *C. diphtheriae* grown under limited aeration conditions in microtitre wells. CRM197 expression was poorly sensitive to repression by $Fe^{3+}$ and was not significantly affected by addition of 1ppm or 2ppm $Fe^{3+}$. Only at 3ppm did a significant drop in CRM197 expression occur. Even at this level of $Fe^{3+}$, the expression of CRM197 was still 79% of that achieved with no addition of $Fe^{3+}$.

Table 7 Corynebacterium diphtheriae expressing CRM197

| Fe3+ added (ppm) | Optical density 46h | pH | CRM (%) |
|---|---|---|---|
| 0 | 18,1 | 7,66 | 100 |
| 200 ppb | 17,9 | 7,72 | 96 |
| 300 ppb | 16,9 | 7,77 | 97 |
| 400 ppb | 16,9 | 7,8 | 106 |
| 500 ppb | 17,2 | 7,83 | 109 |
| 600 ppb | 17,7 | 7,81 | 112 |
| 700 ppb | 17,2 | 7,77 | 109 |
| 800 ppb | 17,2 | 7,77 | 103 |
| 900 ppb | 16,5 | 7,79 | 101 |
| 1ppm | 16,9 | 7,75 | 96 |
| 2ppm | 17 | 7,79 | 88 |
| 3ppm | 16,9 | 7,83 | 79 |

CRM197 yields are expressed as a percentage of the yield achieved without addition of extra $Fe^{3+}$.

DT expression results are shown in Table 8 for C. *diphtheriae* grown in microtitre wells under the conditions indicated. DT production under limited aeration conditions was also poorly sensitive to repression by $Fe^{3+}$. DT expression increased with increasing $Fe^{3+}$ concentration with maximum DT production achieved at 700ppb. Expression of DT started to drop only when the concentration of $Fe^{3+}$ was increased to 3ppm.

Table 8 Corynebacterium diphtheriae expressing Diphtheria Toxin

| Fe3+ added (ppm) | Optical density 46h | pH | DT (%) |
|---|---|---|---|
| 0 | 4,16 | 8,66 | 100 |
| 200 ppb | 4,72 | 8,42 | 106 |
| 300 ppb | 4,6 | 8,47 | 107 |
| 400 ppb | 4,9 | 8,51 | 125 |
| 500 ppb | 4,22 | 8,59 | 155 |
| 600 ppb | 4,48 | 8,51 | 148 |
| 700 ppb | 4,04 | 8,63 | 167 |
| 800 ppb | 4,28 | 8,52 | 164 |
| 900 ppb | 4,58 | 8,62 | 168 |
| 1 ppm | 4,48 | 8,64 | 166 |
| 2ppm | 4,7 | 8,68 | 176 |
| 3ppm | 4,2 | 8,68 | 141 |

DT yields are expressed as a percentage of the yield achieved without addition of extra $Fe^{3+}$.

**Claims**

1. A fermentation process comprising a fermentation step of growing a strain of *Corynebacterium diphtheria* in medium in a fermenter under conditions of agitation sufficient to maintain a homogenous culture and limited aeration such that pO2 within the culture falls to less than 4% for the majority of the fermentation step wherein the fermentation step takes place in either a) a 100-250 litre fermenter at a KLa of 30-60 h-1 or b) in a 250-800 litre fermenter at a KLa of 60-150h-1.

2. The fermentation process of claim 1 wherein the pO2 falls to approaching zero for the majority of the fermentation step.

3. The process of claim 1 or 2 wherein the pO2 of less than 4% is maintained from the time when the *Corynebacterium diphtheria* has grown to a density sufficient for pO2 to fall to less than 4% due to rapid consumption of oxygen, until the fermentation step is completed.

4. The process of any one of claims 1-3 wherein the fermentation step is carried out at constant KLa.

5. The process of any preceding claim wherein the fermentation step is carried out at constant agitation speed and aeration rate.

6. The process of any one of claims 1-3 wherein the fermentation step is carried out under variable kLa conditions.

7. The process of claims 1-6 wherein the fermentation step takes place with an airflow of compressed air of 15-25 Umin and an agitation speed of 150-250 rpm.

8. The process of any preceding claim wherein the medium is CY, SOC or similar medium and pH within the fermenter is held between 7.0 and 7.8 by the degree of aeration without requiring addition of acid or base.

9. The process of any preceding claim wherein the strain of *Corynebacterium diphtheria* produces diphtheria toxin or a mutant thereof, in particular CRM197.

10. The process of any preceding claim wherein the strain of *Corynebacterium diphtheria* is ATCC39255.

11. The process of any preceding claim wherein the medium contains between 10 - 4000ppb of iron.

12. A process for manufacturing a preparation of an antigen from C. *diphtheriae* comprising the steps of carrying out the fermentation process of any one of claims 1-11 and isolating the antigen from C. *diphtheriae* from the culture.

13. The process of claim 12 wherein the antigen from *C. diphtheriae* is diphtheria toxin or a fragment or a mutant thereof (for example CRM197).

14. The process of any one of claims 11-12 comprising a step of adding one or more additional antigen(s) to the antigen from C. *diphtheriae.*

15. The process of claim 14 further comprising a step of conjugating the diphtheria toxin or a fragment or mutant thereof to one or more additional antigen(s).

**Patentansprüche**

1. Fermentationsverfahren, umfassend einen Fermentationsschritt zum Wachsen eines Stammes von Corynebacterium diphtheria im Medium in einem Fermenter unter Bedingungen des Rührens, das ausreichend ist, um eine homogene Kultur und begrenzte Belüftung aufrecht zu erhalten, so dass p02 innerhalb der Kultur auf weniger als 4% für den Hauptteil des Fermentationsschrittes fällt, worin der Fermentationsschritt in (a) einem 100-250 1 Fermenter bei einem KLa von 30-60 h-1 oder (b) in einem 250-800 1 Fermenter bei einem KLa von 60 bis 150 h-1 stattfindet.

2. Fermentationsverfahren nach Anspruch 1, worin p02 für den Hauptteil des Fermentationsschrittes fällt, unter Annäherung von 0.

3. Verfahren nach Anspruch 1 oder 2, worin pO2 von weniger als 4% von der Zeit, wenn das Corynebacterium diphtheria zu einer Dichte gewachsen ist, die ausreichend ist für pO2 zum Fallen auf weniger als 4% aufgrund eines schnellen Sauerstoffverbrauches, bis zur Vollendung des Fermentationsschrittes aufrechterhalten wird.

4. Verfahren nach einem der Ansprüche 1 bis 3, worin der Fermentationsschritt bei einem konstanten KLa durchgeführt wird.

5. Verfahren nach einem der vorhergehenden Ansprüche, worin der Fermentationsschritt bei einer konstanten Rührgeschwindigkeit und Belüftungsrate durchgeführt wird.

6. Verfahren nach einem der Ansprüche 1 bis 3, worin der Fermentationsschritt unter variablen KLa-Bedingungen durchgeführt wird.

7. Verfahren nach den Ansprüchen 1 bis 6, worin der Fermentationsschritt mit einem Luftfluss von komprimierter Luft von 15 bis 25 l/min und einer Rührgeschwindigkeit von 150 bis 250 Upm stattfindet.

8. Verfahren nach einem der vorhergehenden Ansprüche, worin das Medium CY, SOC oder ein ähnliches Medium ist und der pH innerhalb des Fermenters zwischen 7,0 und 7,8 durch den Belüftungsgrad ohne das Erfordernis der Zugabe einer Säure oder Base gehalten wird.

9. Verfahren nach einem der vorhergehenden Ansprüche, worin der Stamm von Corynebacterium diphtheria Diphtherietoxin oder eine Mutante davon, insbesondere CRM197 erzeugt.

10. Verfahren nach einem der vorhergehenden Ansprüche, worin der Stamm von Corynebacterium diphtheria ATCC39255 ist.

11. Verfahren nach einem der vorhergehenden Ansprüche, worin das Medium zwischen 10 und 4.000 ppb Eisen enthält.

12. Verfahren zur Herstellung eines Präparates eines Antigens von C. diphtheriae, umfassend die Schritte des Durchführens des Fermentationsverfahrens nach einem der Ansprüche 1 bis 11 und Isolieren des Antigens von C. diphtheriae von der Kultur.

13. Verfahren nach Anspruch 12, worin das Antigen von C. diphtheriae Diphtherietoxin oder ein Fragment oder eine Mutante davon (zum Beispiel CRM 197) ist.

14. Verfahren nach einem der Ansprüche 11 bis 12, umfassend einen Schritt der Zugabe von einem oder mehreren zusätzlichen Antigenen zum Antigen von C. diphtheriae.

15. Verfahren nach Anspruch 14, weiterhin umfassend einen Schritt zum Konjugieren des Diphtherietoxins oder eines Fragmentes oder einer Mutante davon an ein oder mehrere zusätzliche Antigene.

**Revendications**

1. Procédé de fermentation comprenant une étape de fermentation consistant à cultiver une souche de *Corynebacterium* diphtheriae dans un milieu dans un fermenteur dans des conditions d'agitation suffisante pour maintenir une culture homogène et d'aération limitée telle que La $pO_2$ au sein de la culture chute à moins de 4 % pour la majorité de l'étape de fermentation où l'étape de fermentation prend place dans soit a) un fermenteur de 100 à 250 litres à un KLa de 30 à 60 h-1 soit b) un fermenteur de 250 à 800 litres à un KLa de 60 à 150 h-1.

2. Procédé de fermentation selon la revendication 1, dans lequel la $pO_2$ chute à approximativement zéro pour la majorité de l'étape de fermentation.

3. Procédé selon la revendication 1 ou 2, dans lequel la $pO_2$ inférieure à 4 % est maintenue à partir du moment lorsque *Corynebacterium diphtheriae* s'est développé jusqu'à une densité suffisante pour que la $pO_2$ chute à moins de 4 % à cause d'une consommation rapide de l'oxygène, jusqu'au terme de l'étape de fermentation.

4. Procédé selon l'une quelconque des revendications 1 à 3, dans lequel l'étape de fermentation est réalisée à un KLa constant.

5. Procédé selon l'une quelconque des revendications précédentes, dans lequel l'étape de fermentation est réalisée à une vitesse d'agitation et un taux d'aération constants.

**6.** Procédé selon l'une quelconque des revendications 1 à 3, dans lequel l'étape de fermentation est réalisée dans des conditions variables de KLa.

**7.** Procédé selon l'une quelconque des revendications 1 à 6, dans lequel l'étape de fermentation prend place avec un déplacement d'air comprimé de 15 à 25 l/min et une vitesse d'agitation de 150 à 250 tr/min.

**8.** Procédé selon l'une quelconque des revendications précédentes, dans lequel le milieu est CY, SOC ou un milieu similaire et le pH au sein du fermenteur est maintenu entre 7,0 et 7,8 par le degré d'aération sans nécessiter l'addition d'acide ou de base.

**9.** Procédé selon l'une quelconque des revendications précédentes, dans lequel la souche de *Corynebacterium diphtheriae* produit de la toxine diphtérique ou un mutant de celle-ci, en particulier CRM197.

**10.** Procédé selon l'une quelconque des revendications précédentes, dans lequel la souche de *Corynebacterium diphtheriae* est ATCC39255.

**11.** Procédé selon l'une quelconque des revendications précédentes, dans lequel le milieu contient entre 10 et 4 000 ppb de fer.

**12.** Procédé de fabrication d'une préparation d'un antigène issu de C. *diphtheriae* comprenant les étapes consistant à réaliser le procédé de fermentation selon l'une quelconque des revendications 1 à 11 et à isoler l'antigène issu de *C. diphtheriae* à partir de la culture.

**13.** Procédé selon la revendication 12, dans lequel l'antigène issu de *C. diphtheriae* est la toxine diphtérique ou un fragment ou un mutant de celle-ci (par exemple CRM197).

**14.** Procédé selon l'une quelconque des revendications 11 et 12, comprenant une étape consistant à ajouter un ou plusieurs antigène(s) supplémentaire(s) à l'antigène issu de *C. diphtheriae.*

**15.** Procédé selon la revendication 14, comprenant en outre une étape consistant à conjuguer la toxine diphtérique ou un fragment ou un mutant de celle-ci à un ou plusieurs antigène(s) supplémentaire(s).

A

**Oxygenation profile**

B

**KLa (20/80)**

$y = -17.681x + 5.4021$
$R^2 = 0.9719$

Figure 1

> **Working Seed**
>
> $1.1 \times 10^{10}$ CFU/mL

$5 \, \mu l \, (5 \times 10^7 \text{ CFU})$

> **Preculture flask**
> 3L flask with 500 ml FCD003AA
> 34.5°C, 250 rpm
> →O.D.$_{650}$: 4.0 to 6.0
> (expected between 16-19h incubation)

400 ml

> **Batch fermentation**
>
> Medium        Culture medium (100 L), initial pH~7.4
>                   46 g irradiated antifoam SAG 471
>
> Conditions:     Temperature 34.5°C
>                   KLa= 40 h-1
>                       -Aeration rate: 23 NL/min of air
>                       -Agitation fixed: 240 rpm
>                   Overpressure: 0.5 bar
>                   pH increase limited at 7.8 with $H_3PO_4$ 25%
>
> Duration:      Stop: after minimum 20 h of fermentation
>                   and when pO2 increases to 10%

> **Cooling at 20°C**

> **Microfiltration /Diafiltration**
> Permeate is recovered

> **Sterilising filtration**
> on 0.22 μ membrane

> **Transfer to purification**

Figure 2

Figure 3

Time (hours)

Figure 4A

Figure 4B

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4709017 A
- US 4950740 A
- WO 9324148 A
- WO 9700697 A
- WO 02055105 A
- US 5843711 A
- US 5601827 A
- US 5917017 A
- US 4925792 A
- US 5614382 A
- US 5863891 A
- US 6455673 B
- WO 0050449 A
- US 6342224 B
- WO 9315760 A
- US 4365170 A, Jennings
- US 4673574 A, Anderson
- EP 0161188 A
- EP 208375 A
- EP O477508 A
- WO 9605859 A, A. Cyanamid
- WO 9006951 A, Paton
- WO 9903884 A, NAVA
- US 5804193 A, Briles
- WO 9709994 A, Briles
- WO 97141151 A
- WO 9951266 A
- WO 9640928 A
- EP 0837130 A
- EP 0834568 A
- WO 9818931 A
- WO 9818930 A
- US 9930390 W
- WO 930686 A
- EP 586266 A
- WO 9203467 A
- US 5912336 A
- WO 9306861 A
- WO 9931132 A
- WO 9629412 A
- EP 9902766 W
- WO 0023595 A
- EP 9903603 W
- EP 9906718 W
- WO 9631618 A
- WO 9201460 A
- EP 9805117 W
- WO 9802547 A
- EP 9905989 W
- EP 9908315 W
- WO 9957280 A
- EP 0000135 W
- US 5766608 A
- US 5843464 A
- US 4235877 A, Fullerton

### Non-patent literature cited in the description

- **Moskaug et al.** *Biol. Chem.,* 1989, vol. 264, 15709-15713
- **Germanier.** Bacterial Vaccines. Academic Press, 1984
- **Lingood et al.** *Brit.J. Exp. Path.,* 1963, vol. 44, 177
- **Uchida et al.** *Nature New Biology,* 1971, vol. 233, 8-11
- **Pappenheimer.** *Ann Rev, Biochem.,* 1977, vol. 46, 69-94
- **Rappuoli.** *Applied and Environmental Microbiology,* September 1983, 560-564
- **Rappuoli et al.** *Biotechnology,* February 1985, 161-163
- **Nikolajewski et al.** *J. Biological Standardization,* 1982, vol. 10, 109-114
- **Sambrook J et al.** Molecular Cloning: A Laboratory Manual. Cold Spring Harbor Laboratory Press, 1989
- **Uchida et al.** *J. Biol. Chem.,* 1973, vol. 218, 3838-3844
- **Nicholls ; Youle.** Geneticaly Engineered Toxins. Maecel Dekker Inc, 1992
- **Chu C. et al.** *Infect. Immunity,* 1983, vol. 245, 256
- **Mitchell et al.** Comparison of pneumolysin genes and proteins from Streptococcus pneumoniae types 1 and 2. *Nucleic Acids Res.,* 11 July 1990, vol. 18 (13), 4010
- **Mitchell et al.** Expression of the pneumolysin gene in Escherichia coli: rapid purification and biological properties. *Biochim Biophys Acta,* 23 January 1989, vol. 1007 (1), 67-72

- **Berry ; Paton.** Sequence heterogeneity of PsaA, a 37-kilodalton putative adhesin essential for virulence of Streptococcus pneumoniae. *Infect Immun,* December 1996, vol. 64 (12), 5255-62
- *Infect. Immun.,* 1996, vol. 64, 3544
- **Sanchez-Beato et al.** *FEMS Microbiol Lett,* 1998, vol. 164, 207-14
- **Schryvers et al.** *Med. Microbiol.,* 1999, vol. 32, 1117
- **R. Gluck.** *Vaccine,* 1992, vol. 10, 915-920
- **Takahashi et al.** *Nature,* 1990, vol. 344, 873-875
- The subunit and adjuvant approach. Vaccine Design. Plenum Press, 1995
- **A. Pappenheimer.** *Nature New Biol.,* 1971, vol. 233, 8-11